# EUROPÄISCHE PATENTSCHRIFT

(11) **EP 0 405 132 B1**
(45) Veröffentlichungstag und Bekanntmachung des Hinweises auf die Patenterteilung: **07.09.1994**
(21) Anmeldenummer: 90109556.2
(22) Anmeldetag: 19.05.1990
(51) Int. Cl.: B23Q 5/04, A61B 17/16

(54) **Winkelaufsatz**
Angle drive unit
Unité d'entraînement angulaire

(30) Priorität: 14.06.1989 CH 222/89
(43) Veröffentlichungstag der Anmeldung: 02.01.1991
(73) Patentinhaber: Synthes AG, Chur, CH-7002 Chur (CH)
(72) Erfinder: Frigg, Robert, CH-7270 Davos-Platz (CH); Gisin, Paul, CH-4437 Waldenburg (CH); Scandella, Guido, CH-7494 Wiesen (CH)
(74) Vertreter: Lusuardi, Werther Giovanni, Dr.

(56) Entgegenhaltungen:
- EP-A- 0 167 719
- EP-A- 0 253 526
- EP-A- 0 296 872
- WO-A-84/01099
- DE-A- 2 300 120
- US-A- 4 072 084
- US-A- 4 722 336
- US-A- 4 808 185

## Beschreibung

Die Erfindung bezieht sich auf einen Winkelaufsatz für eine Bohrmaschine gemäss dem Oberbegriff des Anspruchs 1.

Bei einer Vielzahl von osteosynthetischen Verfahren in der Chirurgie werden Bohrmaschinen verwendet, insbesondere um Gewindebohrungen für Knochenschrauben anzubringen und für die Markraumbohrung. Bei letzterer muss jedoch wegen der anatomischgeometrischen Verhältnisse zusätzlich ein an die Bohrmaschine kuppelbares Winkelgetriebe benutzt werden. Solche als Winkelaufsätze konzipierte Winkelgetriebe sind schon seit längerer Zeit bekannt, so offenbart beispielsweise die WO-A-84/01099, die den nächst Kommenden Stand der Technik bildet, ein Winkelstück für zahnärztliche Zwecke, welches jedoch - wie für solche Geräte üblich - ausschliesslich aus metallischen Teilen besteht.
Um die Genauigkeit der mittels solcher Bohrmaschinen (mit oder ohne Winkelaufsatz) plazierter Bohrungen zu verbessern, wurde in den letzten Jahren eine Technik entwickelt, bei welcher der Knochen im Strahlengang eines Röntgengerätes unter Bildverstärkerkontrolle bearbeitet wird. Eine weitere Verbesserung dieser Technik konnte durch den Einsatz diverser in den Strahlengang einzubringender Zielgeräte erreicht werden, welche dazu dienen während des Operationsvorganges die Position und die Bohrrichtung des Bohrers zu überprüfen und in kontrollierter Weise zu verändern.

Ein solches Zielgerät ist beispielsweise in der EP-A- 0 167 719 beschrieben, bei welchem jedoch nur ein einziges Teil - der Aufnahmkopf für den Bohrer - aus einem die Röntgenstrahlung durchscheinenden Werkstoff besteht, so dass nur ein sehr beschränkter Ausschnitt des Operationsfeldes sichtbar wird. Als röntgenstrahlendurchlässige Werkstoffe werden ganz allgemein Kunststoffe vorgeschlagen, ohne Angabe weiter Kriterien, welche für den vorgesehen Zweck von Bedeutung sind (Sterilisierbarkeit und Selbstschmierung). Ein weiterer Nachteil dieses bekannten Zielgerätes liegt, wegen seiner offenen Bauweise, in der hohen Verschmutzungsempfindlichkeit. Trotz der teilweisen Sichtbarkeit des Operationsfeldes bei diesem bekannten Zielgerät lässt sich die Position des Bohrers während des eigentlichen Bohrvorganges nur schlecht kontrollieren, weil die Bohrmaschine und die übrigen metallischen Teile der Vorrichtung das Sichtfeld des Bildverstärkers verdecken. Diese Abdeckung des Gesichtsfeldes hat noch den weiteren Nachteil, dass die automatisch erfolgende Strahlendosierung am Bildverstärker die Strahlenintensität erhöht, um auch die metallischen Teile zu durchdringen. Die nun um einiges erhöhte Strahlenintensität trifft natürlich auch die Hand des Chirurgen, der die Bohrmaschine führen muss. Von besonderer Gefährlichkeit ist dabei die durch Reflexion am Metall auftretende Sekundärstrahlung. Durch die höhere Strahlenintensität ergibt sich auch noch ein weiterer Nachteil, indem jene Stellen im erzeugten Bild, auf die es klinisch wirklich ankommt, nicht mehr oder zu wenig deutlich zu sehen sind.

Hier will die Erfindung Abhilfe schaffen. Der Erfindung liegt die Aufgabe zugrunde, einen Winkelaufsatz für eine Bohrmaschine zu schaffen, welcher röntgenstrahlendurchlässig ist, so dass lediglich der Bohrer im Bildverstärker sichtbar wird und dadurch die Position des Bohrers und dessen Bohrrichtung laufend überprüft und gegebenenfalls korrigiert werden kann unter Beibehaltung einer normalen Strahlenintensität.

Die Erfindung löst die gestellte Aufgabe mit einem Winkelaufsatz, welcher die Merkmale des Anspruchs 1 aufweist.

Die durch die Erfindung erreichten Vorteile sind im wesentlichen darin zu sehen, dass dank des erfindungsgemässen Winkelaufsatzes der Chirurg unbehindert unter Bildwandler-Kontrolle arbeiten kann. Das unter Röntgenbestrahlung auf dem Bildschirm sichtbare Operationsfeld wird nicht wie bei Geräten nach dem Stand der Technik abgedeckt oder abgedunkelt. Ein weiterer Vorteil ergibt sich durch den Umstand, dass die meisten im Operationssaal verwendeten Bildverstärker eine automatische Strahlungsdosier-Vorrichtung besitzen; diese Automatik ermittelt die minimal benötigte Strahlendosis, die benötigt wird, um das im Strahlenfeld befindliche "Objekt" zu durchdringen. Befinden sich nun metallische Teile (z.B. eine handelsübliche Bohrmaschine) im Strahlenfeld, versucht die Automatik des Bildverstärkers diese zu durchdringen. Die daraus resultierende Strahlenerhöhung ist massiv und belastet sowohl den Chirurgen wie auch den Patienten. Neben der direkten Strahlenbelastung im unmittelbaren Strahlengang bewirkt die sogenannte Streustrahlung eine zusätzliche Belastung für den Chirurgen. Diese Streustrahlung tritt vor allem durch Reflexion der Primärstrahlung durch strahlenundurchlässige Objekte auf. Es ist deshalb ein weiterer Vorteil der Erfindung, dass dadurch die Streustrahlung auf ein Minimum reduziert werden kann.
Da der verwendete Bohrer selber aus Metall besteht, d.h. strahlenundurchlässig ist, wird er auf dem Bildschirm des Bildverstärkers deutlich sichtbar. Dank der Erfindung kann somit ohne störende Effekte laufend die Positionierung der Bohrerspitze und die Bohrerrichtung kontrolliert und gegebenenfalls korrigiert werden.
Bei der bevorzugten 90°-Anordnung des erfindungsgemässen Winkelaufsatzes ergibt sich ein weiterer Vorteil, wegen seiner guten Handhabbarkeit im eingeschränkten Raum zwischen Patient und dem Bildverstärker-Empfänger; zudem kann die Lage des kuppelbaren Winkelaufsatz-Gehäuses so gewählt werden, dass sich die Hand des Chirurgen ausserhalb des Strahlenganges des Bildverstärkers befindet.

Das Merkmal der Selbstschmierung des verwendeten röntgenstrahlendurchlässigen Materials ist für die Erfindung wesentlich, weil diese in geschlossener Bauweise realisiert ist, welche eine externe Schmierung nicht zulässt. Auch das Merkmal der Sterilisierbarkeit ist für die Erfindung von Bedeutung, da nicht-sterilisierbare Materialien bald zu deren Verzug und zur Unbrauchbarkeit der Vorrichtung als Ganzes führen würde.

Ein Ausführungsbeispiel der Erfindung, welches zugleich das Funktionsprinzip erläutert, ist in der Zeichnung dargestellt und wird im folgenden näher beschrieben.

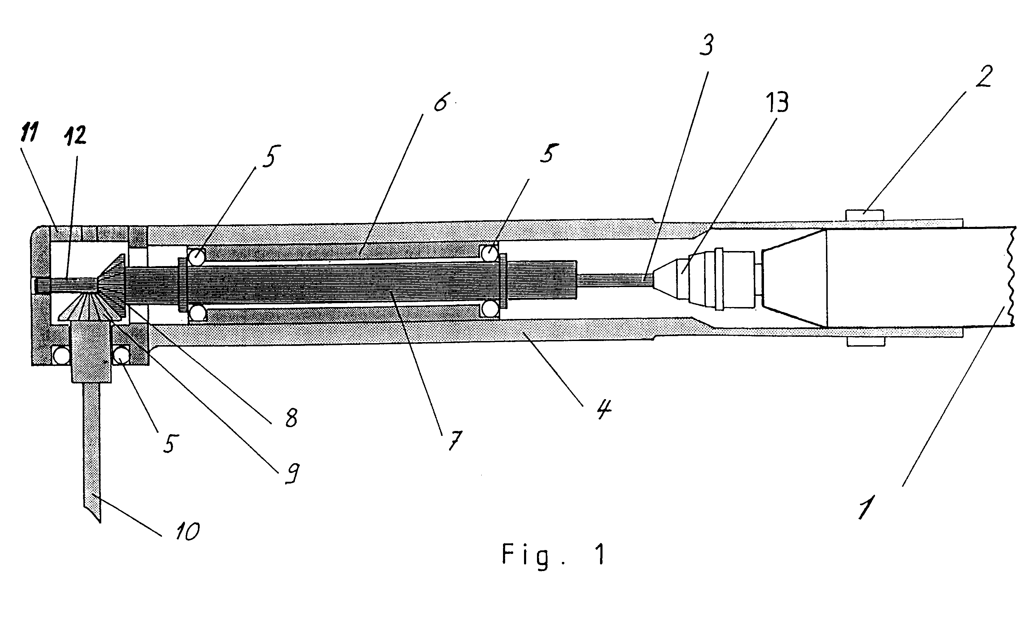

Fig. 1 stellt einen Längsschnitt durch den erfindungsgemässen Winkelaufsatz dar.

Der in Fig. 1 dargestellte Winkelaufsatz besteht im wesentlichen aus dem eigentlichen Winkelgetriebe 7,8,9, welches in einem an die Bohrmaschine 1 kuppelbaren Gehäuse 4 untergebracht ist. Sämtliche Teile des Winkelaufsatzes, mit Ausnahme des zur Kupplung an die Bohrmaschine 1 dienenden Sperringes 2 und die wellenartige Kupplung 3, sind aus einem röntgenstrahlendurchlässigen Material gefertigt. Als dazu geeignete Materialien haben sich insbesondere Polyaetheraetherketone (PEEK), Polyamidimide (z.B. TORLON) und Polyoxymethylen (POM) erwiesen. Diese Materialien sind nicht nur röntgenstrahlendurchlässig, sondern auch selbstschmierend und bis 140 °C sterilisierbar, was für den vorgesehenen Verwendungszweck von grosser Wichtigkeit ist. Auch röntgenstrahlendurchlässige Komposit-Kunststoffe, welche vorzugsweise faser-, gewebe- oder kugelverstärkt sein können, sowie spezielle keramische Werkstoffe können eingesetzt werden.

Die mechanische Funktion des eigentlichen Winkelgetriebes 7,8,9 ist im übrigen durchwegs konventionell. Der rotative Antrieb der Bohrmaschine 1 wird über die wellenartige Kupplung 3, welche im Spannfutter 13 der Bohrmaschine 1 befestigbar ist, auf die Antriebswelle 7 des Winkelaufsatzes übertragen. Die Antriebswelle 7 ist in einem Lagerkäfig 6 untergebracht, welcher ein vorderes und ein hinteres Lager 5 für die Antriebswelle 7 aufweist. Statt der beiden ringförmigen Lager 5 kann die Antriebswelle 7 auch als Gleitlager ausgebildet sein.

Die Antriebswelle 7 trägt an ihrem vorderen Ende ein Kegelrad 8, welches mit einem im 90°-Winkel angeordneten zweiten Kegelrad 9 korrespondiert, welches seinerseits den Bohrer 10 antreibt. Das Kegelrad 9 kann entweder fest mit dem Bohrer 10 verbunden sein oder damit mittels einer (nicht dargestellten) geeigneten Kupplung verbunden sein.
Die Antriebswelle 7 wird zusätzlich durch einen koaxialen Abstützbolzen 12 stabilisiert. Dieser Abstützbolzen 12 ist jedoch nicht unbedingt erforderlich und kann durch eine (nicht dargestellte) Verlängerung des eingesetzten Bohrers 10 bis zum Gehäusedeckel 11 ersetzt werden. Bei dieser Variante kann ein hohler Bohrer verwendet werden, wenn eine Bohrung über einen Spickdraht zu erfolgen hat. Der Spickdraht hat die Funktion einer Sonde, die nach eventuell mehreren Versuchen optimal plaziert wird und den Bohrer 10 beim eigentlichen Bohrvorgang führt. Diese Bohrtechnik wird, z.B. an der Wirbelsäule oder am Becken angewendet, wo das Setzen von Knochenschrauben ausserordentlich präzise durchgeführt werden muss. Trotz der Führung durch den Spickdraht, wird der Aufbohrvorgang unter Bildverstärkerkontrolle durchgeführt; der Grund dafür ist die Gefahr des Vorschiebens des Spickdrahtes und die seitliche Penetration des Bohrers 10 aus dem Knochen. Bei diesem Bohrvorgang, insbesondere an der Wirbelsäule, ist die Röntgenstrahlendurchlässigkeit des Winkelaufsatzes ausserordentlich hilfreich, weil damit keine Verdunkelung oder Überstrahlung des Bildverstärker-Bildes erfolgt, so dass einerseits der zu bohrende Wirbelkörper gut sichtbar bleibt und anderseits der Bohrvorgang kontrollierbar bleibt.

## Patentansprüche

1. Winkelaufsatz für eine Bohrmaschine mit einem, ein hinteres, motorseitiges Ende und ein vorderes, bohrerseitiges Ende aufweisenden Winkelaufsatzgehäuse (4), in welchem eine über eine Kupplung (3) motorisch antreibbare Antriebswelle (7), eine Lagerung (5) und ein Lagerkäfig (6) für die Antriebswelle (7), sowie ein Winkelgetriebe (8,9) angeordnet sind, dadurch gekennzeichnet, dass das Winkelaufsatzgehäuse (4), die Lagerung (5), der Lagerkäfig (6), die Antriebswelle (7) und das Winkelgetriebe (8,9) mindestens teilweise aus einem für Röntgenstrahlen durchlässigen Material bestehen, welches selbstschmierend und bis 140 °C sterilisierbar ist.

2. Winkelaufsatz nach Anspruch 1, dadurch gekennzeichnet, dass das Winkelgetriebe (8,9) aus zwei miteinander im Eingriff stehenden Kegelrädern besteht.

3. Winkelaufsatz nach Anspruch 2, dadurch gekennzeichnet, dass die Antriebswelle (7) durch einen koaxialen, aus einem für Röntgenstrahlen durchlässigen Material bestehenden Abstützbolzen (12) stabilisiert ist.

4. Winkelaufsatz nach Anspruch 2 oder 3, gekennzeichnet durch einen zusätzlichen, am kegelradseitigen Ende des Winkelaufsatzgehäuses (4) angebrachten Deckel (11) aus einem für Röntgenstrahlen durchlässigen Material.

5. Winkelaufsatz nach einem der Ansprüche 1 bis 4, dadurch gekennzeichnet, dass das für Röntgenstrahlen durchlässige Material aus verstärktem Polyaetheraetherketon PEEK oder Polyamidimid besteht.

6. Winkelaufsatz nach einem der Ansprüche 1 bis 4, dadurch gekennzeichnet, dass das für Röntgenstrahlen durchlässige Material aus verstärktem Polyoxymethylen (POM) besteht.

7. Winkelaufsatz nach einem der Ansprüche 1 bis 6, dadurch gekennzeichnet, dass das bohrerseitige Kegelrad (9) des Winkelgetriebes (8,9) fest mit einem Bohrer (10) verbunden ist.

8. Winkelaufsatz nach einem der Ansprüche 1 bis 7, dadurch gekennzeichnet, dass die Lagerung (5) aus mindestens zwei Kugellager oder 1 - 2 Gleitlager besteht.

9. Winkelaufsatz nach einem der Ansprüche 1 bis 8, gekennzeichnet durch einen im Winkelaufsatz integrierten über die Kupplung (3) wirkenden Antrieb (1), vorzugsweise in Form einer Pressluftturbine oder eines Elektromotors.

## Claims

1. An angular attachment for a surgical drilling machine with an angular attachment housing (4) having a rear end adjacent to the motor and a front end adjacent to the drill, said angular attachment housing (4) having arranged therein a drive shaft (7) motor-drivable by means of a coupling shaft (3), a bearing (5) and a bearing cage (6) for the drive shaft (7), as well as an angular gear (8,9), characterized in that the angular attachment housing (4), the bearing (5), the bearing cage (6), the drive shaft (7) and the angular gear (8,9) are consisting at least partially of a material penetrable by X-radiation and which is self-lubricating and sterilizable up to 140°C.

2. An angular attachment according to claim 1, characterized in that the angular gear (8,9) consists of two intermeshing conical gear wheels.

3. An angular attachment according to claim 2, characterized in that the drive shaft (7) is stabilized by means of a coaxial support bolt (12) made of a material penetrable by X-radiation.

4. An angular attachment according to claim 2 or 3, characterized by an additional cover (11) made of a material penetrable by X-radiation and positioned at that end of the angular attachment housing (4) adjacent to the conical gear wheels.

5. An angular attachment according to claims 1 - 4, characterized in that the material penetrable by X-radiation consists of reinforced polyetheretherketone (PEEK) or polyamideimide.

6. An angular attachment according to claims 1 - 4, characterized in that the material penetrable by X-radiation consists of reinforced polyoxymethylene (POM).

7. An angular attachment according to claims 1 - 6, characterized in that the conical gear wheel (9) of the angular gear (8,9) which is adjacent to the drill is firmly connected with the drill (10).

8. An angular attachment according to claims 1 - 7, characterized in that the bearing (5) consists of at least two ball bearings or 1 to 2 sliding bearings.

9. An angular attachment according to claims 1 - 8, characterized by a drive unit (1) integral with the angular attachment and acting over the coupling shaft (3), said drive unit (1) preferably being in the form of an air turbine or an electromotor.

## Revendications

1. Dispositif de renvoi d'angle pour une foreuse, comportant un boitier (4) de dispositif de renvoi d'angle présentant une extrémité arrière située côté moteur, et une extrémité avant située côté foret, avec disposés dans ce boîtier (4) un arbre d'entraînement (7) pouvant être entraîné par le moteur par l'intermédiaire d'un accouplement (3), un système de paliers (5) et une cage à paliers (6) pour l'arbre d'entraînement (7), ainsi qu'une transmission coudée (8, 9), caractérisé en ce que le boîtier (4) du dispositif de renvoi d'angle, le système de paliers (5), la cage à paliers (6), l'arbre d'entraînement (7) et la transmission coudée (8, 9) sont constitués au moins partiellement d'un matériau transparent aux rayons X, qui est autolubrifiant et peut être stérilisé jusqu'à 140°C.

2. Dispositif de renvoi d'angle selon la revendication 1, caractérisé en ce que la transmission coudée (8, 9) est constituée de deux roues coniques s'engrenant l'une dans l'autre.

3. Dispositif de renvoi d'angle selon la revendication 2, caractérisé en ce que l'arbre d'entraînement (7) est stabilisé par une tige d'appui (12) constituée d'un matériau transparent aux rayons X.

4. Dispositif de renvoi d'angle selon la revendication 2 ou 3, caractérisé par un couvercle supplémentaire (11) en un matériau transparent aux rayons X, installé à l'extrémité, située côté roues coniques, du boîtier (4) du dispositif de renvoi d'angle.

5. Dispositif de renvoi d'angle selon l'une des revendications 1 à 4, caractérisé en ce que le matériau transparent aux rayons X est constitué de polyéther éthercétone (PEEK) ou de polyamidimide renforcés.

6. Dispositif de renvoi d'angle selon l'une des revendications 1 à 4, caractérisé en ce que le matériau transparent aux rayons X est constitué de polyoxyméthylène (POM) renforcé.

7. Dispositif de renvoi d'angle selon l'une des revendications 1 à 6, caractérisé en ce que la roue conique (9) de la transmission coudée (8, 9), située côté foret, est solidaire d'un foret (10).

8. Dispositif de renvoi d'angle selon l'une des revendications 1 à 7, caractérisé en ce que le système de paliers (5) est constitué d'au moins deux paliers à billes ou de 1 - 2 paliers à glissement.

9. Dispositif de renvoi d'angle selon l'une des revendications 1 à 8, caractérisé par un entraînement (1) intégré au dispositif de renvoi d'angle et agissant par l'intermédiaire de l'accouplement (3) et présentant, de préférence, la forme d'une turbine à air comprimé ou d'un moteur électrique.
